# EUROPEAN PATENT APPLICATION

(11) **EP 2 163 196 A2**
(43) Date of publication of application: **17.03.2010**
(21) Application number: 09014944.4
(22) Date of filing: 27.12.2005
(51) Int. Cl.: A61B 5/053

(54) **Device for measuring the presence of foreign substances in a living organism and for individually selecting preparations for increasing an organism's life activity**

(30) Priority: 31.12.2004 RU 2004138996; 01.07.2005 RU 2005120494
(62) Divisional of application: 05851132.0
(71) Applicant: Gotovsky, Mikhail Yurievich, Moscow 123242 (RU)
(72) Inventor: Gotovsky, Mikhail Yurievich, Moscow 123242 (RU)
(74) Representative: Gibler, Ferdinand

(57) **Abstract**

The invention concerns to area of pharmacy, biological physics and biodiagnostics, more exactly to development of preparations having ability of magnetic-resonance exposure on micro- and macro organisms with the purpose of activization and protection of their vital activity in conditions of ecological environment at the expense of individual selection of such preparations with the help of the special device. The problem of stability of exposure on biological object with the simple and very habitual for use, universalised, portable, marked if necessary, agent of activation of vital activity of biological object at an ecological level is not solved in any known development. Therefore the first task of the present invention is the creation of universal portable preparation for activation of vital activity of biological object with the help of the physiological carrier capable to be marked, to work in a wide range of an electromagnetic field (EMF) and to keep stable initial properties for a long time during storage. Other task is the creation of a similar preparation capable to work in a wide spectrum of a variable magnetic field (VMF). Both types of preparations are capable to activate the vital activity of organisms, intensifying energetic activity of organism, including accelerating elimination of the harmful foreign agents. Those or other preparations are chosen with the help of the magnetic resonance device capable to find out the foreign agent in the organism being investigated.

## Description

### FIELD OF APPLICATION

Discovery refers to pharmacy, biological physics and biodiagnostics, more precisely - to development of preparations, having capacity of magnetic-resonance impact upon microorganisms and macroorganisms with the view of activation and protection of their vital activity under environmental conditions at the expense of individual selection of such preparations using a special apparatus.

Great majority of civilized countries population lives nowadays under severe, ecologically strenuous conditions. Frequently, different unfavorable agents and foreign compounds, which the given organism can use neither for energy production, nor for building of living cell constituents, nor for protection of the organism from adverse environmental effect, are present in the environment in a varying degree. Agents, polluting environment - emissions from industrial enterprises, cars' exhaust, waste from animal production units, different sprays, fertilizers, pesticides, detergents, food and cosmetic conserving agents and dyes, pathogen particles, which are alien to this ecosystem, e.g. introduced viruses, high-molecular and low-molecular allergy-causing agents, as well as some types of medicines, drugs, tobacco smoke, nicotine, heavy metals etc., get into the organism not only because they are connected with their voluntary application by this organism, but simply because under present circumstances: with food, water, air, contacting with other organisms etc. Detection of various foreign agents in the organism is time-consuming and intrusive for the patient in most cases; selection of acceptable supporting and protective medicines from these "invisible enemies" is difficult and almost non-personalized. As to investigation of the internal organism biofields with the view of individual selection of such medicines, application of protective preparations, charged with physical fields (i.e. having magnetic-resonance effect when entering the organism), as corrective or vital activity stimulating, is not only painless and noninvasive, but also practically exclusive internal universal protective medicines from many foreign agents.

### PRIOR ART

In living organisms, numerous physical and biochemical reactions and interactions take place, which are strictly coordinated among themselves with respect to time and space. All this order is directed to permanent self-preservation and self-reproduction of organisms under certain external conditions. Any biological object or organism is an informatively integrated system, in which all its elements, as well as cells, tissues and organs interact. Interaction among different elements of a biological object or parts of a living organism and the environment is subject to information fields, due to which mutual control is performed. Information fields, having influence on the organism, can be divided into internal and external ones. Each of these field types can be divided in to 3 groups again.

The following fields refer to internal ones: internal information fields, inherent in a healthy organism and performing control and synchronization in it with a view of searching for and sustaining homeostasis, physiological fields; internal information fields of disturbances, disturbing processes of control and synchronization in the organism and causing loss of control over homeostasis, pathological fields; internal fields of noises, hindering processes of control over homeostasis, but not leading to its significant disturbance.

The following fields refer to external ones: external information fields, used by the organism for production of signals, controlling its homeostasis; external information fields of disturbances, disturbing processes of control over homeostasis in the organism; external fields of noises, hindering processes of control over homeostasis of the organism.

Electromagnetic oscillations, being generated by different systems of the organism with a view of searching for and sustaining its homeostasis, as well as providing required adaptive responses, serve as an example of internal fields of type I. Electromagnetic oscillations, resulting from diseases (introduction of viruses, bacteria, toxins, etc.) and hindering production of signals by the organism, required for searching for and returning to homeostasis, serve as an example of internal fields of type II. Internal noises of the organism, hindering, but not disturbing control serve, as a matter of fact, as an example of internal fields of type III.

Biorhythmicity, as well as most methods of therapy, used in modem medicine, e.g. allopathy, homeopathy, reflexotherapy, physiotherapy, etc., serve as an example of internal fields of type I. Effect of external causes such as ecological toxins, external electromagnetic fields, etc., serves as an example of internal fields of type II. Cosmic radiation serves as an example of internal fields of type III.

Known are methods for registration of electromagnetic fields and radiations on special media, which are in different phase states at the moment of record, attempted with a view to asses the state of biological objects, which are produced for example in VIS (GB # 515866), IR or RF (DE # 3605104) range on photofilms and other field-sensitive media.

Known are methods of exposure with electromagnetic fields and radiations in the wide range of frequencies for correction of the biological objects' state, for example stimulation of some human functions (US # 5095901, US 5617856, 1997, US 6032063, 2000, RU 2154508, 2000), activation of plant productivity (SU # 1665952) or yeast cells (SU Nº 1592328).

Known are methods of the fields' exposure to different biological objects, both for vertebrate organisms (RU 2105575, 1998, RU 2121334, 1998) and for invertebrate organisms, for example, germs (RU 2146540, 2000). However, most part of known effects on biological objects is performed by way of large and small structures, sometimes unhandy and complex enough, whose effect permanently changes and is often accompanied by hidden or not enough studied by-effects.

An analogue, close to the claimed discovery, is the research work, in which a method of bioresonanse therapy (patent RU 2065297, 1996) is described, where effect of a biological field with electromagnetic nature on the organism is used. Known are different methods for detection of foreign agents in the organism, consisting in instrumental chemical analysis (chromatography, spectroscopy, and the like) of blood samples, urine samples and other metabolic products of a living organism [1]. However, carrying-out of such analyses is connected with great labor intensity and significant time consumption, including obtaining and preparation of samples, as well as the necessity of sampling either just after a foreign agent has entered the organism or a few hours or, at the outside, a few days. Furthermore, methods of chemical analysis don't offer the possibility to detect the presence of a foreign agent in the organism retrospectively, for example, after a few weeks or months, and measuring equipment is not distinguished by portability or performance.

Study of patent information showed that diagnostic examination of patients using special automated (particularly, bioresonanse) apparatus became very actual in recent years (patents of the Russian Federation # 2070405. 1996, # 2070406, 1996). Known are also different electrophysiological devices for examination of the organism's state by electrical conductivity of skin in the range of acupuncture points (refer, for instance, patent RU # 2137458, 1999).

Effect of such physical field, as magnetic, relatively long used in biology and medicine, may be reputed as sufficiently studied. It is used for biological, scientific, agricultural, and physiotherapeutic purposes (refer, for instance, [1,2,3,])

In recent years, there appeared preparations, generally used for magnetic-resonance (MR) influence at the expense of external influence of some magnet-containing materials on the organism (refer, for instance, patent US 6148225, 2000, patent US 6157281, 2000).

But these are objects, which are complex enough for implementation and many-valued with respect to effects.

An analogue, close to the claimed discovery, is the research work, in which a complex for MR-therapy is described, where effect of a physical field with magnetic nature on the organism with application of a cell made of ferromagnetic or analogous materials is used (patent US 6150911, 2000).

At the present time, remote control of the dosage, injected from the syringe (refer patent US # 5792117, 1998), as well as remote monitoring of the patient's state by way of determination of required marker parameters in the organism (patent US # 6270455, 07.08.2001) became possible due to automated devices connected to a computer.

However, known adaptive bioinformation methods and devices don't allow to determine the presence of a foreign agent in the organism, such as pesticide, herbicide, anabolic preparation, semisynthetic antibiotic, allergen, microbiological particles, drugs, nicotine, etc., which is present in the organism at a very low concentration (including homeopathic level).

As a research work, which is closest to the claimed discovery, may be considered the work, disclosed in patent RU # 1745100, 1987, where the functioning of an apparatus for diagnostics of the organism's state by examination of the state of biologically active points is described. Detection of a foreign agent (particularly, toxic diphteroid) by way of skin conductivity measurement at biologically active points (BAP) is described in the discovery. In the presence of a foreign agent, skin conductivity in the area of BAP is changing.

But this method doesn't allow determining presence of foreign agents subject to environmental impact inside the living organism in vivo, as well as assessing and selecting such a individually active preparation with magnetic-resonance action, which is able to stimulate vital activity and to protect the organism from harmful agents of the environment.

### BRIEF DESCRIPTION

The discovery refers to pharmacy, biological physics and biodiagnostics, more precisely - to development of preparations, having capacity of magnetic-resonance impact upon microorganisms and macroorganisms with the view of activation and protection of their vital activity under environmental conditions at the expense of individual selection of such preparations using a special apparatus.

The problem of stability of effect on a biological object using a simple and very traditional for application, universalized, portable, and labeled, if necessary, means for stimulation of a biological object's vital activity on ecological level isn't resolved in any known work. Therefore, the first objective of this discovery is development of a universal portable preparation for stimulation of a biological object's vital activity using a physiological carrier, capable to be labeled, to work in a wide range of electromagnetic field (EMF), and to maintain stable initial properties in storage for a long time. Another objective is development of such a means, capable to work in a wide spectrum of variable magnetic field (VMF). Both types of the preparations are able to stimulate organisms' vital activity, enhancing energetic activity of the organism, including enhancing elimination of harmful foreign agents.

Another aspect of this work is development of such an apparatus, which allows determining presence of low, up to trace concentrations of foreign substances in the organism such as pesticides, anabolic substances, allergens, heavy metals' ions, drugs, and other substances polluting environment. On the basis of these data, the apparatus analyses the possibility of use of preparations with magnetic-resonance action, developed by us, for stimulation of the organism's vital activity with a view to protect from harmful agents influence. Technically it results in the fact that using this apparatus it is possible to increase noninvasively sensibility, precision and selectivity of in vivo determination of harmful agents, contained in the human and animal organism at extremely low concentrations (homeopathic level), entering the organism from environment voluntarily or involuntarily; the apparatus permits assessment of the ways for protection from harmful agents using preparations, developed by us, in the given environmental conditions.

Another one object of this discovery is a method for the organism's vital activity stimulation, which includes selection of preparations with magnetic-resonance action. Selection is performed using the apparatus, described in this application, which is able to detect presence of harmful agents in the organism and to select necessary preparations, stimulating vital activity and capable of protection of the organism from harmful agents under conditions of surrounding ecosystem. Preparations are administered in the individual regime.

### Brief description of drawings

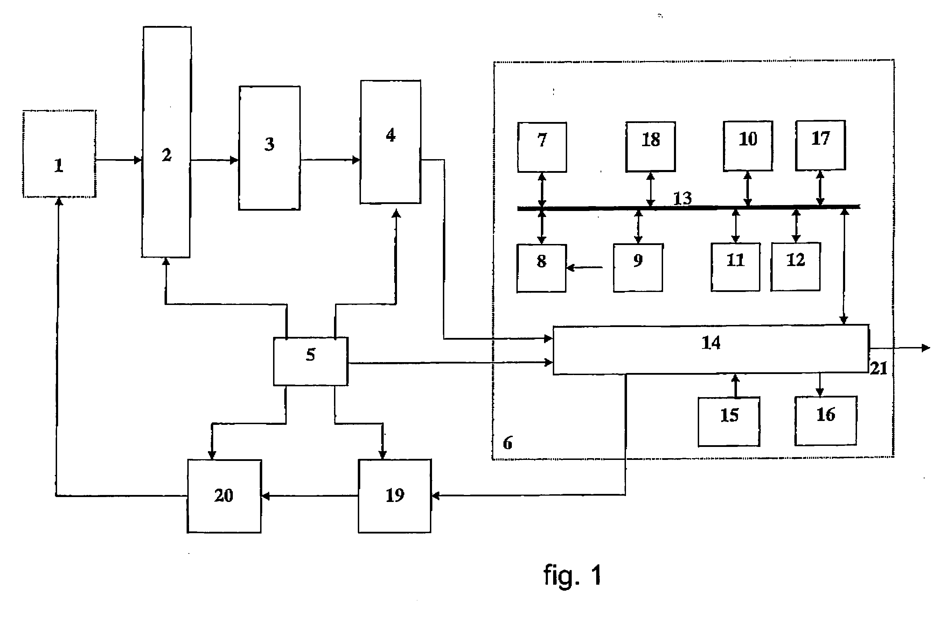

The functional diagram of the device is shown on the drawing (fig.1) with numeration of each functional block. The device allows to determine presence in organisms of low up to trace concentration of foreign substances such as pesticides, ions of heavy metals and other substances polluting environment. Basing on these data the device performs analysis of possibility to use developed by us preparations of magnetic-resonance action for stimulation of vital activity of organism for protection from influence of harmful agents. Besides the device has wide range of other valuable properties listed in detailed description of the invention.

### THE USE OF THE INVENTION

We have developed preparations with magnetic-resonance action for stimulation of a biological object's vital activity with a view of its protection from influence of foreign agents, voluntarily or involuntarily entering the organism from the environment. Such preparation contains a carrier, treated with electromagnetic field (EMF); the carrier is thereby a substance, selected from a group: pharmaceutically acceptable solid basis, containing not less than 23% weight of crystalline substance, physiological saline or liquid crystals, and the carrier was treated with EMF at 0.01Hz-9.0 kHz, 10.0-100.0 kHz or 0.5-10.0 MHz during 0.5 - 60 minutes.

As a crystalline substance, preparation contains inorganic salt of potassium, magnesium, calcium or sodium, saccharine, glucose, fructose, maltose, rhamnose, ribose, dextrose or their mixture.

*The discovery is explained with following examples of the new preparation.*

### EXAMPLE 1: Preparation, activated with EMF, on a powdered carrier.

In order to make the preparation, a powder, consisting of mixture of pharmaceutically acceptable ingredients - talc, sodium lactate, and sodium chloride is taken in the following ratio (% weight):
Sodium chloride - 25,0
Sodium lactate -10,0 talc - residual.

The powder is put inside the loop, capable for generating EMF, for instance electromagnetic (EM) loop, described in patent RU # 2070406, developed by author of this discovery, and exposed to EMF radiation at 10.0-100.0 kHz during 6.0 minutes. Preparation is able to keep EM-properties during 12 months at room conditions.

### EXAMPLE 2. Preparation, activated with EMF, on a tablet carrier.

In order to make the preparation, a tablet, containing potassium chloride, potato starch, additional substances: gelatin, polyethylene glycol, iron oxide, yellow sunset dye is taken in the following ratio (weight %):
Potassium chloride - 30,0
Polyethylene glycol - 10,0
Other adjunct - 5.0
Starch - residual.

Tablets with weight 0.6 -1.0 g are put inside the loop, as described in example 1, at 0.5-10 MHz and held 10-30 minutes. Shelf-life - 5 years, guaranteed shelf-life - 25 months.

### EXAMPLE 3. Preparation, activated with EMF, on a physiological saline.

A few vials, containing physiological saline of sodium chloride, are put inside the loop, capable for generating EMF at 0.01Hz-9.0 kHz for 60-70 sec., saline is sterilized and sealed into vials. Preparation keeps properties during 12 months.

### EXAMPLE 4. Preparation, activated with EMF, on a diluted culture medium.

Eagle's culture medium is diluted with water in ratio 1:7, filled into 10 ml vials, sterilized, and sealed. Sealed vials are put inside the loop, capable of generating EMF, and held at 0.01Hz-9.0 kHz during 2 minutes. The preparation obtained keeps new properties, acquired after EMF-activation, during 12 months.

Method for obtaining the preparation, described above, is performed by way of EMF exposure to carrier, the carrier is thereby a substance, selected from a group: pharmaceutically acceptable powdered or tablet basis, containing not less than 23 weight % of crystalline substance, physiological saline or liquid crystals; and exposure to EMF is performed at 0.01Hz-9.0 kHz, 10.0-100.0 kHz or 0.5-10.0 MHz during 0.5 - 60 minutes. Mixture of not less than 2 substances, selected from the following group - talc, starch, dextrane, starch syrup, magnesium oxide, colloid silicon dioxide, cellulose or its derivatives, lactose or its derivatives - is used as powdered or tablet basis.

As crystalline substances, preparation contains inorganic salt of potassium, magnesium, calcium or sodium, saccharose, glucose, fructose, maltose, lactose, rhamnose, ribose, dextrose or their mixture are used as crystalline substances. Additional substances are also used for tabletting.

Pharmaceutically acceptable dyes are used for labeling the degree of EMF activation of the carrier. For instance, a carrier without dye may be used for identification of activation degree for a solid carrier on a tabletted basis; a carrier without dye may be used for EMF at 0.01Hz-9.0 kHz; a yellow dye may be added for activation degree of a tabletted carrier of EMF at 10.0 - 100.0 kHz, and for EMF at 0.5 -10.0 MHz - a red dye.

Physiological saline of sodium chloride or culture medium, diluted with water in ratio 1: (5-10) are used as a physiological fluid; medium is selected from the following group: Ringer's solution, Eagle's, Bordet-Gengou's, Levintal's, Fidles', Zeissler's medium, serum agar or peptone broth. When using a powdered or tablet basis, optimal EMF exposure is within 6.0 - 30.0 minutes. When using liquid crystals, optimal EMF exposure is within 1.0 - 5.0 minutes. When using a physiological saline, optimal EMF exposure is within 0.5 - 2.0 minutes.

Another preparation, relating to this application, is a preparation for stimulation of the biological object's vital activity, containing a carrier, treated with variable magnetic field (VMF), distinguished by the fact that the carrier is a substance, selected from the following group - pharmaceutically acceptable solid basis, containing salt of monovalent, bivalent, or three-valence metal or their mixture, physiological liquid basis or liquid crystals; the carrier is treated with VMF, having flux density of 1.8 - 8.0 µT, using a frame inductor, or VMF, having flux density of 0.13 - 1.50 mT, using solenoid inductor. Such a preparation has as a solid basis a mixture of not less than 2 substances, selected from the following group - talc, starch, dextrane, starch syrup, colloid silicon dioxide, cellulose or its derivatives, lactose or its derivatives; it may also contain additional substances for tabletting, pharmaceutically acceptable dyes for labeling of the solid carrier activation degree. Preparation, activated with VMF, contains a physiological saline of sodium chloride or a culture medium, diluted with drinking water in ratio 1: (2-10), selected from the following group - Eagle's medium, Ringer's solution, Bordet-Gengou's, Levintal's, Fidles', Zeissler's medium, serum agar or peptone broth - as a physiological fluid.

Preparations obtained in this way were used for stimulation of the organism's vital activity, which is proved by the following examples.

### Example 5.

Investigations were performed under experimental conditions on commercially available microorganisms, used in agriculture as producers of protein, amino acids and other food substances, used in animal production, as well as in food production, connected with fermentation process. In particular, testing of the preparation, described in example 3 (EMF-activation) or activated with one of the methods using VMF, was performed on the following strains-producers of glutaminic acid: *Mycrococcus glutamicus, Brevibacterium flavum, Br. aminogenes, Br. saccharalyticum*. During their cultivation under standard conditions, 1 ml of preparation was added to 10 ml of culture medium: in the reference, preparation without its activation with EMF or VMF was used, and cultivation was performed without applying preparation. The final product yield in the experiment group was 17 - 25% higher than in the reference.

### Example 6.

In experiments, carried out by North-Caucasian research institute for animal production of Russian Academy of Agricultural Sciences (Krasnodar, Russia), it was determined a directed change of metabolism in commercial farming fowl (ducks, geese), which consumed with food the preparation, described in example 1, as a veterinary preparation. Comparative analysis showed statistically reliable increase in animals' productivity in the experiment group in comparison with the reference group, which had the powdered carrier, not activated with EMF, and the group, which didn't have the preparation; calculated increase was within 35-40 % and more. Investigation with the preparation, activated with VMF, in the same ecological system, all other conditions of handling and recording of check measurements in animals being equal, showed that calculated increase in fowl productivity was only 25,7±1,6 (p<0,05). This fact indicates the prior use of preparations, activated with EMF, in relatively ecologically clean systems.

### Example 7.

Investigation was performed on healthy volunteers: 6 man aged 23 to 54 and 8 women aged 25 to 56.

Proving the discovery in volunteers is based on a known physiological thesis on basal metabolic rate, characterizing amount of energy, which is released in the organism at vital activity stimulation, for instance, by change in volumes of absorbed oxygen and disengaged carbon-dioxide gas. Resulting energy is accepted as heat energy and expressed in kilocalories [4].

Basal metabolic rate was determined in patients when seated at rest by way of graphic recording of changes in volumes of absorbed oxygen and disengaged carbon-dioxide gas at closed respiratory system using spirograph «Metatest-2». Basal value amounted to 36,5 J/m²s - 42.2 J/m²s for men, and to 30,1 - 41,8 J/m²s for women. Patients received preparation described in example 2, as well as other preparations, obtained according to the claimed method, containing saccharose, fructose, or ribose as a crystalline substance at different levels of preparation activation with EMF. Same preparations without EMF activation were used for reference. Level of basal metabolic rate authentically increased in all patients, at the average by 5 - 8 % to the reference (p<0,05); and subjectively, health and spirits of patients became better. Furthermore, additional testing of preparations showed that such preparations can be used for patients, containing foreign agents in the organism because of impact of unfavorable environment and other circumstances.

Additional testing of preparations, obtained according to the claimed method, showed that their biological activity and EM-properties are kept in 99.7% of cases upon expiration of 12 months since the date of their making.

Another aspect of this work is development of such an apparatus, which allows determining presence of low, up to trace concentrations of foreign substances in the organism, such as drugs, pesticides, anabolic substances, allergens, heavy metals' ions, and organic and inorganic atmospheric contaminants against operation of different plants, technogenic catastrophes and nature cataclysms.

Technically, it results in the fact that using this device it is possible to increase - by a noninvasive express-method - sensibility, precision and selectivity of in vivo determination of harmful agents, voluntarily or involuntarily entering the human and agricultural animal's organism at different ecological circumstances, including force-majeure.

For determination of the foreign agent it is necessary to reveal the special biologically active point (BAP) in the organism, so-called reproducible point. The presence of the foreign agent is determined by electrical conductivity in this point. Moreover, the additional information about determined foreign agents, in form of their spectral characteristics corresponding to various dilutions down to homeopathic level, so-called test-indicator, is entered into a measuring circuit of our bioresonance device. As the test-indicator the records of the spectral characteristics of various ecologically significant substances recognized to be harmful for human organism, are used (see **tab. 1, 2,3**) [5].

During the interaction of the wave spectra of the «external foreign agent», taken from the test-indicator, with another one, present in organism (sought) the phenomenon of a resonance occurs, that finds the reflection in change of measurable parameters of electromagnetic signals emitted by the organism (e.g., ECG, EEG etc.). We have established that the most convenient way is the measuring-in of dynamics of skin electrical conductivity changes in acupuncture points or skin BAP. For measurements both absolute and relative parameters consisting in change of skin conductivity in a certain direction (its decrease or increase) could be used. Such measurements allow to reveal the presence of the foreign agent in the organism. In case of its presence in the organism, the skin electrical parameters measured in BAP before and after comparison with the agent-marker from the test-indicator, situated in the external measuring circuit, differ from each other. Otherwise these changes are absent. The measurements can be carried out in any BAP or acupuncture point, connected to organ which is not having pathological disturbances, i.e. reproducible point of measurements, which is revealed by a so-called method of «pumping». The basis of way of determination of the foreign agent in the organism is the vegetative resonant test (VRT). In the beginning the selection of BAP or acupuncture point in the patient is carried out, which point is designated as a point of measurement (MP), choosing among them the reproducible MP. The reproducible point is MP, in which the repeated measurements by method of «pumping» show the same measurable values. In our case the so-called «dual point of measurement» is selected, which is obtained with the help of probe with two vertical cross sections under angle 90 degrees.

Method of «pumping» is carried out as follows:
- pressure or other active exposure is carried out on chosen MP, using the test probe, supplied by an elastic plate;
- duration of pumping process should not exceed 3-5 seconds;
- for people the pressure of the test probe onto a point should not exceed 100-200 g (1-2 Newtons);
- after the achievement of the maximal magnitude of measurable value in MP, for example, 40 standard units (s.u.) of scale, the pressure of the test probe is decreased without separation of the tip of the test probe of the active electrode from the point (at this time values of parameters by the device's scale are decreasing). Then the pressure of the test • probe has been again gradually resume ("pumping"), If the initial value of 40 s.u. of the scale is achieved again after that exposure, then the MP is considered reproducible; if not, it indicates the presence of some pathology in organs and systems connected to this MP. Such point is considered non-reproducible and can not be used for measurements;
- the pressure should be smooth through time (shockless and without spasmodic change of pressure). If the exposure on MP is too strong, it could become unsuitable for measurement.

To increase resolution of the method, in the equipment realizing the invention the expansion of measuring scale gauge up to 80 s.u. is used, regardless of the initial level of reproducible MP, obtained for conducting of research.

After finding the reproducible MP and expansion of the scale of the device up to 80 s.u. the test preparation, which marks presence or absence of the foreign agent in the patient's organism, is introduced into the measuring circuit. Then the repeated measurement by the method of «pumping» is carried out. If the value received on the device scale is less than 80 s.u., the test is considered positive ("yes", there is the foreign (harmful) agent in the organism); if it comes back to the initial level - the test is considered negative ("no", there was not the fact of entry of the foreign agent into the organism).

On the drawing (fig.1) the functional diagram of the device is presented, containing the block 1 «electrodes», block 2 «switching», block 3 «amplification», analog-digital converter 4, block 5 «management» and block 6 «measurement and registration» including the random access memory (RAM) 7, block 8 «comparison of parameters», controller of individual exposure in the form of test probe with two vertical cross sections under angle 90 degrees 9, block 10 «information storage», in which the wave characteristics of various foreign agents (test-indicator) are registered, processor 11, timer 12, data bus 13, input/output interface 14, control panel 15, visual display unit 16, «calibration» block 17, read-only memory 18, buffer register 19, digital-to-analog converter 20 and bus 21 connection with the external drive and PC.

The work of the device is carried out as follows.

Before the beginning of examination the device is switched on and the control of its functioning and self-testing of the block 6 are carried out according to the algorithm, included in it, using the "calibration" block 17.

After calibration of the device the doctor passes on to a mode of diagnostics, for which the measurement of parameters of electroconductivity of skin cover in BAP area is carried out. For this purpose from the control panel 15 the programmed selection of necessary electrodes pair (passive and active) is carried out. The indifferent (passive) electrode is given by doctor to the patient for holding it in a hand, and the active electrode - is supplied to an acupuncture point for generation of an analog measurable signal. The active electrode is made in the form of test probe, representing as an elastic plate, to which the resistive-strain sensor is pasted on. During the exposure of the test probe on skin surface the value of force or other effect is recorded by the resistive-strain sensor and can be measured as an electrical signal.

As it is necessary to provide in measured BAP the certain exposure of the test probe (for example, effort of 1-2 N, relative to the area of the test rod end face), the operator from the control panel gives a command for connection of controller of exposure of the test probe 9 and timer 12, which is fixed for the certain period of time (3-5 sec). At achievement of exposure of the test probe on the acupuncture point, appropriate to time given during determined interval, the block 8, «comparison of parameters», disconnect the controller of exposure and timer. The obtained analog signal after its amplification in the block 3 and digital transformation in the analog-digital converter 4 through the input/output interface 14 and data bus 13 is recorded into RAM 7 and arrives at the processor 11 for processing. The signal is processed according to the included program and is shown as the graphic image on the visual display unit 16.

At the achievement of steady condition of this image (i.e. when the indications of values of electroconductivity repeat reiteratedly, that is the evidence of reproducibility of the given acupuncture point) operator, using the control panel 15, starts the program, according to which the command is given, which through the input/output interface 14 goes to the connection of the appropriate test-indicator from the block of «information storage» 10. in which the wave characteristics of every foreign agent in the defined dilution (including homoeopathic) are recorded, to the block 1 «electrodes» (through the output of the input/output interface 14, buffer register 19 and digital-to-analog converter 20).

If after exposure on the acupuncture point of the test-indicator connected to it, the indications on the visual display unit 16 do not change, it indicates the absence of patient's reaction on the given agent, i.e. the given agent in the patient's organism is absent. Then operator gives a command to connect the next test-indicator (from a set of substances summarize in tables 1-3) etc.

Run over of the tests-indicators is conducted until occurrence of resonance, the reaction on which is shown in change of parameter of electroconductivity. It attests that the given foreign agent is present in the patient's organism.

As the contents of the RAM 7 are copied into the read-only memory 18, all the data in the device are kept. Through the bus 21 of the input/output interface 14 they could be transferred to an external network or to the PC. Through the same bus the information about the test-indicator could be supplemented and edited. Besides, the device has additional capabilities providing possibility to research condition of the whole organism and its organs that allows to give recommendations for selection of magnetic-resonance preparations for reliefing patient's condition, especially under conditions of polluted environment. The invention is illustrated by following examples.

### Example 8.

The examination of a contingent of the persons in the Krasnodar Territory working with agents used in agriculture for protection of plants (pesticides and herbicides) has been carried out. These agents are used for spraying of rice and wheat fields, and also gardens and kitchen gardens. As a result of measurements in 130 people having contact with pesticides and herbicides, their presence in human organisms was revealed in 99.3 % in comparison with the control group of persons, who were not connected with these chemicals by the nature of their activity. The further research has shown that a number of volunteers (about 100 people) have expressed a wish to carry out trial intake of our preparations of magnetic-resonance action, activated by both electromagnetic field (EMF) and variable electromagnetic field (VEF). The results of such researches, including those carried out in territories with other ecological conditions, are shown in the **table 4.**

The preparations developed by us, keeping their biological properties for a long time during storage, have appeared highly effective in protection of organism from the influence of the various ecologically harmful agents. The activation of vital activity of organism with the help of our preparations with magnetic-resonance action results not only in intensification of energetic processes in organism, acceleration of metabolic processes of nutritious substances assimilation, but also in starting of processes of elimination of the harmful agents which have entered organism accidentally or by the power of ecological circumstances. Therefore one more object of our project is a way of activation of vital activity of organism, including its inspection and selection of our preparations with magnetic-resonance action with participation of the device characterized above, using the effect of these preparations on organism in conditions of environmental ecosystem. The researches have shown that for organism living in not polluted ecosystem activation of vital activity is mainly carried out by preparations processed with EMF. For organisms living in ecosystem polluted by wastes of agricultural or industrial manufacture, and also of urban agglomerate, it would be better to carry out activation of vital activity by preparations processed by VEF with an induction of 1,8- 8,0 mc Tesla or 0,13 - 1,5 m Tesla.

In the extended experiment healthy volunteers (men and women in the age of from 18 up to 59 years) participated, totally 1738 people from various areas of Russian Federation (Moscow, Novosibirsk, Tver, Oryol Regions. Krasnodar Territory). The patients have been examined in detail using our device for detection in their organisms of the trace quantities of the foreign agents inherent to ecology of the given district (ecological monitoring of the organism), and also for detection of the foreign agents connected to harmful habits of the patient (monitoring of the organism's foreign agents).

The contingent of the persons, in whose organisms the sought foreign agents (from among given in tab. 1-3) have not been found out, has been symbolically designated - ecologically healthy "ECOH".

The contingent of the persons with presence of traces of the agents, characteristic for atmospheric pollution of agricultural wastes, in organism has been designated as "ECOAC".

The contingent of the persons with presence of traces of the agents, characteristic for atmospheric pollution of industrial wastes, in organism has been designated as "ECOIND"

The contingent of the persons with presence of traces of the agents, characteristic for atmospheric pollution of urban agglomerate wastes, has been designated as "ECOUA".

All investigated persons received our preparations within 4 weeks in dose of 2 tablets 3 times per day. The tablets activated by all kinds of processing by physical fields under our technique were used. As the control the tablets which have not been activated by EMF or VEF were used. Such preparations have been designated as NAP.

The active preparations for convenience of results estimation have been symbolically designated as follows:
EMF 1 - activated by EMF with frequency 0,01 Hz - 9,0 kHz
EMF 2 - activated by EMF with frequency 10 -100 kHz
EMF 3 - activated by EMF with frequency 0,5-10 MHz
VEMF 4- activated by VEMF with an induction 1,8 - 8,0 mc Tesla
VEMF 5 - activated by VEMF with an induction 0,13- 1,5 m Tesla.

By the results of research we revealed percentage of positive effects of our preparations. As positive effect the intensification of metabolism more than 5 % (see ex. 7), in comparison with initial level, was considered. In the table 4 the levels of percentage of positive effects of the surveyed contingent are given.

It is visible from the table, that for activation of vital activity of organism under conditions of the not polluted environment, it is advisable to apply preparations processed by EMF under our technology. Under conditions of the polluted environment, both of agricultural or industrial wastes, and also urban agglomerate wastes, using of preparations processed by VEF is more effective.

A Preparation for activation of vital activity of biological object containing carrier processed by an electromagnetic field (EMF). The distinguishing feature of preparation is that the carrier represents the substance chosen from the group: pharmaceutically acceptable solid base containing not less than 23 % by weight of crystal substance, physiological liquid or liquid crystals, and the carrier processing has been performed by EMF with frequency 0,01 Hz - 9,0 kHz, 10,0-100,0 kHz or 0,5-10,0 MHz during 0,5 - 60 minutes.

This preparation distinguished by that it contains as the crystal substance any inorganic salt of potassium, magnesium, calcium or sodium, saccharine, glucose, fructose, maltose, rhamnose, ribose, dextrose or their mixture.

A Preparation for activation of vital activity of biological object containing carrier processed by a variable magnetic field (VEF), the distinguishing feature is that the carrier represents substance chosen from the group: pharmaceutically acceptable solid base containing salt of univalent, bivalent or trivalent metal or their mixture, physiological liquid base or liquid crystals, and the processing of the carrier has been perfomed by VEF with induction of 1,8 - 8,0 mc Tesla with the help of loop inductor or by VEF with induction of 0,13 - 1,50 m Tesla with the help of solenoid inductor.

One of the preparations of above, distinguished by that it contains as a solid base the mixture of not less than 2 substances chosen from the group: talc, starch, dextrin, starch syrup, colloidal silicon dioxide, cellulose or its derivatives, lactose or its derivatives.

One of the preparations of above, distinguished by that it can in addition contain auxiliary substances for tabletting, pharmaceutically acceptable colorants for labeling the degree of activation of the solid carrier.

One of the preparations of above, distinguished by that it contains as a physiological liquid the physiological solution of sodium chloride or nutrient medium diluted by drinking water in the ratio 1:(2-10), chosen from the group: Eagle's medium, Ringer's solution, Bordet-Gengou potato blood agar, Levinthal's medium, Fild's medium, Zeissler's medium, serum agar or beef-extract broth.

The device for individual selection of one of the preparations of above, for activation of vital activity of organism, containing the block of electrodes, analog-digital converter connected to the input of the block of "measurement and registration" including processor, (which inputs-outputs are connected to the data bus, input/output interface, block of the "information storage", block of "calibration", control panel and visual display unit), and also block of management, the distinguishing features of the device are:
the buffer register and digital-to-analog converter are entered into it, the block of electrodes is connected to the analog-digital converter through blocks of switching and amplification of signal, the active electrodes in the block of electrodes are made as elastic plates from metals with different conductivity, supplied by resistive-strain sensors, and into the block of "measurement and registration" the block of comparison of parameters, controller of individual parameter of exposure and timer are superinduced,
at the same time the outputs of the block of management are connected accordingly to managing inputs of the block of switching, anaiog-to-digital converter, buffer register, digital-to-analog converter and input/output interface, the information input of the input/output interface is connected to the output of the analog-digital converter, the output is connected through the buffer register and digital-to-analog converter to the block of electrodes, and inputs-outputs to inputs-outputs of the data bus, to which the inputs-outputs of the blocks of comparison of parameters, storage of the information about the spectral characteristics of the ecologically harmful agents and block of calibration, and also read-only memory and timer are connected,
moreover, the output of controller of individual parameter is connected to the input of the block of comparison of parameters and with the data bus by quantitative and qualitative parameters of ecosystem, and control panel and visual display unit are connected to the input/output interface.

The device, distinguished by that the input/output interface has the connection bus to the external information tank or PC.

A method of activation of vital activity of organism, including its inspection and individual selection of preparations of magnetic-resonance action of above with the help of the device of before, subject to conditions of ecological environment with the subsequent introduction of the chosen preparations in an effective amount.

The method distinguished by that for organism living in not polluted ecosystem, activation of vital activity is mainly carried out by preparations processed by EMF.

The method distinguished by that for organism living in ecosystem polluted by agricultural or industrial wastes, and also urban agglomerate wastes, activation of vital activity is mainly carried out by preparations processed by VEF.

So principally new system was developed which involves compact preparations of magnetic-resonance action, the device for optimal selection of such preparations for certain ecological conditions and method for stimulation of organism's vital activity with said preparations.

**Table 1.**

| Substance | Content in the environment | Entry in human | Threat to humans |
|---|---|---|---|
| Cadmium | In the air, near production plants up to 0.5 mcg/m³, normally 0.02 - 0.05 mcg/m³, in city - 0.02 - 370 mcg/m³, countryside - 0.004 - 0.026 mcg/m³. | With water (AAS 0.01 mg/l), food, air | Proteinuria, diseases of kidneys, osteomalacia, prostate cancer |
| Manganese | Accumulates in the air near production plants and in some objects; linoleum, matches, pyrotechnic goods; air in cities - 10 mcg/m³ | With air | Progressive affections of central nervous system, lethargy, Parkinson's syndrome, pneumonia |
| Copper | In brass electrotechnical goods, ware, chemicals, dyes | With water and food | Intoxication, anemia, hepatitis |
| Molybdenum | In alloys, dyes, glasses, lubricants, in soil in certain areas | With air (AAS 4 - 5 mcg/m³), food, water | Disturbances of central neural system endemic ataxia, gout |
| Nickel | Accumulates in sea organisms, nickel-coated ware, water 1 - 70 mcg/l | With air, food | Bronchial cancer, dermatitis, intoxication, allergy |
| Nitrates and nitrites | In water up to 10 mg/l, in air 1 - 40 mcg/m³, in soil water even more than 300 mg/l | With water (AAS 45 mg/l), food | Metgenoglobinemia |
| Nitrosocompound | In meant and fish treated with nitrites | With water, food | Cancer, mutagenic and teratogenic action |
| Nitric oxide | In air of big cities with intensive car movement | With air | Intoxication, respiratory diseases |
| Mercury | Normally in air up to 0.05 mcg/m³, in sweet water up to 0.2 mcg/l in sea water up to 0.3 mcg/l in polluted water up to 5 mcg/l in dump water up to 50 mcg/l | With water(AAS 0.001 mg/l), air (mercury vapor), food (AAS 0.3 mg per week) | Intoxication, Minamat diseases, paralysis, psychical inferiority of new-borne |
| Lead | Normally in water up to 10 mcg/l, sea water - 7, at the bottom deposits up to 3000 mcg/l, in the city air - 2 - 4 mcg/m³, in the countryside air - up to 0,2 mcg/m³, in the virgin lands 8 - 20 mcg/kg, cultivated lands - up to 300 mcg/kg | With air (AAS 0.01 - 0.2 mcg/m³), water (AAS 0.0 mg/l), food | Intoxication, affection of central nervous system, kidneys, liver, brain, sexual organs |
| Fluorine | In city air 0.05 - 2 mcg/m³, in mcg/m³, in water normally more than 0.5 mg/l | With water, air | Fluorosis, diseases of teeth and bones |
| Chromium | In alloys, dyes, hardeners, heat-resistant bricks | With air | Bronchial cancer |
| Cyanides | Pollute certain food | With water (AAS 0.05 mg/l) | Intoxication |
| Zinc AAs **-** ambient air | In zinc-covered ware, industrial air standard for Russia. | With air (AAS 5 mcg/m³) | Intoxication |

**Table 2.**

| Element | Substance | Atmosphere air AAS, mcg/m³ |
|---|---|---|
| Lead | | 0.003 |
| | Nonorganic compounds | 0.0003 |
| | Sulphide | 0.0017 |
| | Lead-tin solder | - |
| Copper | | - |
| | Oxide | 0.002 |
| | Sulphate | 0.001 |
| | Sulphide 0.001 | |
| | Chloride | 0.001 |
| | Copper-nickel ore | - |
| Cadmium | | |
| | Cadmium and its non-organic compounds | - |
| | Oxide | 0.001 |
| Tin | | |
| | Chloride | 0.05 |
| Mercury | | 0.0003 |
| | Metallic salts | 0.0003 |
| Zinc | | |
| | Oxide | - |
| | Sulphate | - |

| | | |
|---|---|---|
| AAS - ambient air standard for Russia. | | |

**Table 3.**

| Substance | AAS in water according to sanitary-toxicological sign of harm | Danger class |
|---|---|---|
| Acrylamide | 0.01 | 2 |
| Aluminium | 0.5 | 2 |
| Aniline | 0.1 | 2 |
| Acetoneciangidin | 0.001 | 2 |
| Barium | 0.1 | 2 |
| Benzol | 0.5 | 2 |
| Benz(a)perin | 0.000005 | 1 |
| Beryllium | 0.0002 | 1 |
| Boron | 0.5 | 2 |
| Brome | 0.2 | 2 |
| Bismuth | 0.1 | 2 |
| Tungsten | 0.005 | 2 |
| Hexamethylenediamine | 0.001 | 2 |
| Dimethylamine | 0.1 | 2 |
| Dimethyldioxane | 0.005 | 2 |
| 2.5- dichloronitrobenzol | 0.1 | 2 |
| Dichloroethane | 0.02 | 2 |
| Dichloroethylene | 0.0006 | 1 |
| Diethylmercury | 0.0001 | 1 |
| Cadmium | 0.001 | 2 |
| Cobalt | 1.0 | 2 |
| Lithium | 0.003 | 2 |
| Nitrates | 10.0 | 2 |
| Pentachlorobiphenile | 0.01 | 1 |
| Pyridine | 0.2 | 2 |
| Mercury | 0.0005 | 1 |
| Lead | 0.003 | 2 |
| Strontium | 7.0 | 2 |
| Stibium | 0.005 | 2 |
| Thallium | 0.0001 | 1 |
| Tetrachlorobenzene | 0.02 | 2 |
| Tetrachloroethyl | 0.02 | 2 |
| Tetraethyl lead | Not at all | 1 |
| Tricresynphosphate | 0.005 | 2 |
| Trichlorobiphenyl | 0.001 | 1 |
| Fluorine | 1.5 | 2 |
| Chloroform | 0.06 | 2 |
| Carbon tetrachloride | 0.006 | 2 |
| Ethylmercurochloride | 0.0001 | 1 |

| | | |
|---|---|---|
| AAS - ambient air standard for Russia. | | |

**TABLE 4.**

| Preparation/Contingent | ECOH | ECOAC | ECOIND | ECOUA |
|---|---|---|---|---|
| EMF 1 | 86, 2± 5,91 | 75,3 ±5,1 | 78,2±4,6 | 73,6±5,9 |
| EMF 2 | 85,3 ± 5,12 | 78,8±4,3 | 74,6±5,8 | 72,1± 6,9 |
| EMF 3 | 88,5 ±6,16 | 69,9± 5,4 | 72,8± 4,3 | 79,1± 3,8 |
| NAP | 1,8±0,9 | 0 | 2,0 ±0.7 | 0 |
| VEMF 4 | 73,9±5,8 | 82,3±4,3 | 87,7±5,3 | 86, 9±7,5 |
| VEMF 5 | 76,4±7,2 | 80,9±6,7 | 86,4±7,5 | 89,5±6,3 |
| NAP | 1,1±0,8 | 3,2±0,9 | 0 | 2,2±0,6 |

### References

1. A. White et al. «Bases of biochemistry», in three volumes, Moscow, "Mir", 1981.
2. Z.N. Nakhilnitskaya «Magnetic field and ability to live opraHN3MOB», Problem of space biology, v. 37, Moscow, "Science", 1978.
3. L.Ya. Skurikhina, M.A. Shishlo "Magnetotherapy, Balneology and physiotherapy, v.1, Moscow, "«Medicine", 1985, p. 471-484.
4. G.R. Soloviyova «Magnitotherapeutic equipment», Moscow, "Medicine", 1991.
5. B. Andrew «Experimental physiology», Translation from English under ed., M., "Mir", 1974, p. 122-127.
6. S. Delyatitskiy et. al. «The Ecological dictionary», Moscow, 1993

## Claims

1. A device for determining presence in a living organism of low up to trace concentration of foreign substances such as pesticides, ions of heavy metals and other substances polluting environment and for individual selection of preparations for activation of vital activity of the living organism, whereby the preparations are containing a carrier, whereby
either the carrier represents the substance chosen from the group: pharmaceutically acceptable solid base containing not less than 23 % by weight of crystal substance, physiological liquid or liquid crystals, and the carrier is processed by an electromagnetic field (EMF), and the carrier processing has been performed by EMF with frequency 0,01 Hz - 9,0 kHz, 10,0-100,0 kHz or 0,5-10,0 MHz during 0,5 - 60 minutes,
or the carrier represents substance chosen from the group: pharmaceutically acceptable solid base containing salt of univalent, bivalent or trivalent metal or their mixture, physiological liquid base or liquid crystals, and the carrier is processed by a variable magnetic field (VEF), and the processing of the carrier has been performed by VEF with induction of 1,8 - 8,0 mc Tesla with the help of loop inductor or by VEF with induction of 0,13 - 1,50 m Tesla with the help of solenoid inductor,
and whereby the device containing a block of electrodes (1), a block of management (5), and an analog-digital converter (4) connected to an input of a block of "measurement and registration" (6) including a processor (11), which processors (11) inputs-outputs are connected to a data bus (13), an input/output interface (14), a block of "information storage" (10), a block of "calibration" (17), a control panel (15) and a visual display unit (16), **characterized in that**
a buffer register (19) and a digital-to-analog converter (20) are entered into it,
the block of electrodes (1) is connected to the analog-digital converter (4) through a block of switching (2) and a block of amplification (3) of signal,
the active electrodes in the block of electrodes (1) are made as elastic plates from metals with different conductivity, supplied by resistive-strain sensors,
and into the block of "measurement and registration" (6) a block of comparison of parameters (8), a controller of individual parameter of exposure (9) and a timer (12) are superinduced,
at the same time outputs of the block of management (5) are connected accordingly to managing inputs of the block of switching (2), the analog-to-digital converter (4), the buffer register (19), the digital-to-analog converter (20) and the input/output interface (14), an information input of the input/output interface (14) is connected to the output of the analog-digital converter (4), an output of the input/output interface (14) is connected through the buffer register (19) and digital-to-analog converter (20) to the block of electrodes (1), and inputs-outputs of the input/output interface (14) to inputs-outputs of the data bus (13), to which inputs-outputs of the block of comparison of parameters (8), the block of "information storage" (10) for storing information about the spectral characteristics of the ecologically harmful agents, the block of calibration (17), a read-only memory (18) and the timer (12) are connected,
an output of the controller of individual parameter (9) is connected to an input of the block of comparison of parameters (8) and with the data bus (13) by quantitative and qualitative parameters of ecosystem,
and the control panel (15) and visual display (16) unit are connected to the input/output interface (14).

2. The device of claim 1, **characterized in that** the input/output interface (14) has a connection bus (21) to an external information tank or a PC.

3. A method for determining presence in a living organism of low up to trace concentration of foreign substances such as pesticides, ions of heavy metals and other substances polluting environment and individual selection of preparations for activation of vital activity of the living organism, whereby the preparations containing a carrier, whereby
either the carrier represents the substance chosen from the group: pharmaceutically acceptable solid base containing not less than 23 % by weight of crystal substance, physiological liquid or liquid crystals, and the carrier is processed by an electromagnetic field (EMF), and the carrier processing has been performed by EMF with frequency 0,01 Hz - 9,0 kHz, 10,0-100,0 kHz or 0,5-10,0 MHz during 0,5 - 60 minutes,
or the carrier represents substance chosen from the group: pharmaceutically acceptable solid base containing salt of univalent, bivalent or trivalent metal or their mixture, physiological liquid base or liquid crystals, and the carrier is processed by a variable magnetic field (VEF), and the processing of the carrier has been performed by VEF with induction of 1,8 - 8,0 mc Tesla with the help of loop inductor or by VEF with induction of 0,13 - 1,50 m Tesla with the help of solenoid inductor, **characterized in** an inspection and individual selection of the preparations by a device **characterized in** one of claims 1 to 2, subject to conditions of ecological environment with the subsequent introduction of the chosen preparations in an effective amount.

4. The method of claim 3, **characterized in that** for organism living in not polluted ecosystem, activation of vital activity is mainly carried out by preparations processed by EMF.

5. The method of claim 3, **characterized in that** for organism living in ecosystem polluted by agricultural or industrial wastes, and also urban agglomerate wastes, activation of vital activity is mainly carried out by preparations processed by VEF.

6. Use of a device **characterized in** one of claims 1 to 2 for determining presence in a living organism of low up to trace concentration of foreign substances such as pesticides, ions of heavy metals and other substances polluting environment and basing on these data for performing an analysis of possibility to use preparations of magnetic-resonance action for stimulation of vital activity of a living organism for protection from influence of harmful agents, whereby the preparations for activation of vital activity of biological object containing a carrier, and whereby
either the carrier represents the substance chosen from the group: pharmaceutically acceptable solid base containing not less than 23 % by weight of crystal substance, physiological liquid or liquid crystals, and the carrier is processed by an electromagnetic field (EMF), and the carrier processing has been performed by EMF with frequency 0,01 Hz - 9,0 kHz, 10,0-100,0 kHz or 0,5-10,0 MHz during 0,5 - 60 minutes,
or the carrier represents substance chosen from the group: pharmaceutically acceptable solid base containing salt of univalent, bivalent or trivalent metal or their mixture, physiological liquid base or liquid crystals, and the carrier is processed by a variable magnetic field (VEF), and the processing of the carrier has been performed by VEF with induction of 1,8 - 8,0 mc Tesla with the help of loop inductor or by VEF with induction of 0,13 - 1,50 m Tesla with the help of solenoid inductor.
